# EUROPEAN PATENT APPLICATION

(11) **EP 4 309 681 A1**
(43) Date of publication of application: **24.01.2024**
(21) Application number: 23183772.5
(22) Date of filing: 06.07.2023
(51) Int. Cl.: A61L 2/20, A61L 9/015, F21V 33/00

(54) **LAMP WITH INTEGRATED SANIFICATION SYSTEM**

(30) Priority: 08.07.2022 IT 202200014443
(71) Applicant: Enamuna, Amen, 10010 Candia Canavese (TO) (IT); Micheletto, Giovanni, 10010 Canda Canavese (TO) (IT); Advance S.r.l., 10080 San Benigno Canavese (TO) (IT)
(72) Inventor: ENAMUNA, Amen, 10010 CANDIA CANAVESE (TO) (IT); MICHELETTO, Giovanni, 10010 CANDIA CANAVESE ( TO) (IT)
(74) Representative: Boario, Enrico

(57) **Abstract**

Lamp (2) for illuminating at least one space (V) of a building (E), comprising: at least one source (3) adapted to emit light in the visible spectrum for illuminating said space; a power supply circuit (20), adapted to supply power to said source (3); a base (22) adapted to be laid on a surface available in said space; a support structure (24), structurally connected to and extending from the base (22), and adapted to support said source (3); and a lampshade (4) adapted to divert and/or create a soft light in order to reduce the intensity of the light of the source (3) perceivable by an observer. Said lamp (2) comprises a sanification device (5) for sanitizing the surfaces and the air in said space. Said sanification device (5) being integrated into at least a part of said lamp (2) and being powered by said power supply circuit (20). Said sanification device (5) comprising: an ozonizer (6) adapted to generate ozone; a control system (58) adapted to suitably activate said ozonizer device (6) independently of the activation of said source (3) of the lamp (2); and a fan (56). The control system (58) is adapted to activate the fan (56) and the ozonizer (6) in a coordinated manner in order to maximize the distribution of ozone in the space (V). the sanification device (5) comprises an ozone sensor (57) adapted to detect the presence of ozone. The ozone sensor (57) being electrically and electronically connected to said control system (58).

## Description

The present invention relates to a lamp adapted to illuminate a space of a building, e.g. a table lamp, a bedside lamp, a ceiling light fixture, a wall light, a floor lamp, etc., comprising a sanification system adapted to sanitize said space.

Sanification systems are known which can sanitize air by having a flow of air taken in from a space pass through a sanification source and then delivering it back into the space. Sanification systems are also known which are adapted to sanitize surfaces by dispensing a sanitizing substance into the space.

According to the solutions currently known in the art, such systems are stand-alone devices to be placed into the space when required, to be then removed at the end of the sanification phase.

Although these are portable devices, they are nonetheless quite bulky.

The technical problem of sanitizing one or more spaces of a building in an effective, inexpensive and safe manner is known in the art.

Those solutions which are suitable for sanitizing air cannot effectively sanitize surfaces as well. Moreover, in the currently known solutions that make use of UV sources, the latter suffer from the limitation that they will only sanitize those surfaces which are directly illuminated by the UV source. Therefore, any areas or regions not directly illuminated will not be sanitized.

It is also known that in buildings comprising a plurality of separate spaces, e.g. hotel rooms, the use of portable sanitizers makes it difficult to sanitize the whole building, in addition to requiring much time for sanitizing a plurality of spaces, while potentially putting at risk the operators in charge of positioning and removing such portable sanification systems in/from the various rooms of a hotel.

When used for sanitizing buildings comprising a plurality of separate spaces, portable sanitizers are very expensive, and very high costs would have to be incurred should one want to provide each space with a dedicated sanitizer. Moreover, such sanitizers are not pleasant to the eye, and cannot therefore be left in the space during a guest's stay. The use of a plurality of sanitizers also requires that an operator has to take care of the installation and subsequent removal of the sanitizers from the spaces at the end of the sanification procedure. Furthermore, such sanitizers must be appropriately stored when not in use, thus requiring the availability of a secure storage area, and have to be transported from the storage area to the various spaces, and vice versa.

Patent application CN2487951Y discloses an eye protecting lamp for oxygen barroom, adapted to generate ozone and micro negative ion to kill harmful germs in the air to clean the air, thus avoiding eye tiredness due to prolonged use.

Furthermore, patent application WO2021210034A1 discloses an integrated system for sanitization and emergency lighting of rooms, comprising: an external housing, which includes a white light source with an optical system, apt to realize an emergency lighting, a battery for operating said emergency lighting in the absence of a main power supply; a chamber, provided with reflecting walls and internal lens shade baffles, in which a photo-catalytic reactor and/or a UVC light source are housed, and a fan, of axial or tangential type, which creates inside said chamber a circulation or flow of forced air taken from outside, upstream of said chamber through inlet slots, and reintroduced outside, downstream of said chamber through outlet slots, and an electronic control circuit, which controls the operation of the white light source, the UVC light source and/or the photo-catalytic ballast, the battery and the fan. Inside said chamber, an ambient light sensor is housed, aimed at measuring the brightness of the environment in which said system is positioned.

The market is constantly looking for technical solutions wherein it is possible to associate one sanitizer to each space of a building at low cost, without requiring works, e.g. masonry works, for the installation of sanification systems or devices, and wherein the sanification systems or devices are provided as an unobtrusive solution that does not perturb the furnishings of the space.

The present invention aims at solving the above-described technical problems by providing a lamp for illuminating at least one space of a building which comprises a sanification device for sanitizing said space, in particular both the air and the surfaces therein, wherein said sanification device is integrated into at least a part of said lamp.

One aspect of the present invention relates to an illumination lamp having the features set out in the appended claim 1.

Auxiliary features are set out in dependent claims appended hereto.

The features and advantages of the lamp will become clear and apparent in light of the following description of several possible embodiments thereof, provided herein by way of non-limiting example, as well as from the annexed drawings, wherein:
- Figure 1 shows, by way of non-limiting example, one possible embodiment of the lamp according to the present invention, which incorporates a sanification device;
- Figures 2A and 2B show different embodiments of a lamp according to the present invention in two different sectional views, wherein: Figure 2A is a sectional side view of the lamp of Figure 1; Figure 2B shows, in a rotated sectional view, another possible embodiment of a lamp according to the present invention;
- Figure 3 shows a schematic plan view of a building comprising a plurality of spaces, wherein in each space there is at least one lamp according to the present invention.

With reference to the above-listed figures, reference numeral 2 designates as a whole a lamp according to the present invention.

Lamp 2 according to the present invention is adapted to illuminate, at least partly, a space "V" of a building "E". Therefore, lamp 2 according to the present invention is a lamp for illuminating at least one space "V" of a building "E".

Said lamp 2 according to the present invention comprises: at least one source 3, adapted to emit light in the visible spectrum for illuminating said space "V"; and at least one, preferably only one, power supply circuit 20 adapted to supply power to said source 3. Preferably, said source 3 is adapted to emit warm white light.

Said lamp 2 according to the present invention further comprises at least one base 22, adapted to be laid on a surface available in said space "V"; and at least one support structure 24, structurally connected to and extending from base 22, and adapted to support said source 3.

Lamp 2 according to the present invention further comprises at least one lampshade 4, which is adapted to divert and/or create a soft light in order to reduce the intensity of the light of source 3 perceivable by an observer. Preferably, said lampshade 4 is connected, preferably in a removable manner, to said support structure 24; alternatively, said lampshade is self-supporting and/or connected to said base 22.

Said lamp 2 according to the present invention comprises a sanification device 5 for sanitizing said space "V".

Said sanification device 5 is integrated into at least a part of said lamp 2, e.g. into said base 22, said support structure 24 and/or said lampshade 4. Said sanification device 5 is powered by said power supply circuit 20.

Said sanification device 5 according to the present invention comprises: an ozonizer 6 adapted to generate ozone; and a control system 58 adapted to suitably activate said ozonizer 6 independently of the activation of said source 3 of lamp 2.

The present invention makes it possible to associate a sanification device 5 with each space "V" of a building "E" in a simple manner, at low cost, and without requiring any works, e.g. masonry works, for the installation of said sanification device 5 in a space "V" of a building "E". Furthermore, the present invention makes it possible to keep the furnishings of space "V" of building "E" unchanged, since said sanification device 5 is incorporated into lamp 2, thus being camouflaged in the furniture of space "V".

The present invention makes it also possible to maximize the sanification and disinfestation effect, since it offers high performance, while being quite inexpensive. In addition, the present solution ensures an effective sanification of both the air and the surfaces, even those not directly visible.

In a preferred, but merely illustrative and non-limiting, embodiment of lamp 2 according to the present invention, said sanification device 5 comprises: at least one inlet 52, adapted to allow air to enter; and at least one outlet 54, adapted to allow ozone-containing air, in particular air containing ozone produced by said ozonizer 6, to exit. Said ozonizer 6 is positioned between said inlet 52 and said outlet 54. It is apparent that, in this embodiment, said ozonizer 6 is located in a place where it is not accessible to an observer. Such a solution performs both a technical function, by preventing any tampering, and an aesthetic function, since said ozonizer 6 is not visible to an observer, thus providing an aesthetically discreet and non-invasive solution.

Preferably, said inlet 52 and said outlet 54 are formed in the structure of lamp 2. The very structure of lamp 2 permits creating a duct between said inlet 52 and said outlet 54, through which air can flow.

In an even more preferable embodiment of lamp 2 according to the present invention at least one fan 56 is comprised, said fan 56 being preferably comprised in said sanification device 5. Said control system 58 is adapted to activate said at least one fan 56 and said ozonizer 6 in a coordinate manner in order to maximize the distribution of ozone in said space "V". Said fan 56 is suitably powered by said power supply circuit 20.

Said fan 56 is preferably a high-performance one, thus speeding up the distribution of ozone in space "V".

For example, said fan 56 can move 100 m³/h to 400 m³/h of air, e.g. 300 m³/h of air. Preferably, said fan 56 is a single-rotor fan.

Said fan 56 is preferably located near inlet 52, for the purpose of maximizing the air moving capacity. Preferably, said fan 56 is arranged in a housing comprised in the structure of said inlet 52.

In a preferred, but merely illustrative and non-limiting, embodiment of said lamp 2 according to the present invention, said inlet 52 comprises at least one removable filter 521.

Preferably, said inlet 52 defines a housing in which said filter 521 can be inserted, which can be easily removed for maintenance, cleaning or replacement.

Said filter 521 is, for example, a paper, cell, soft-pocket, stiff-pocket, HEPA and/or activated charcoal filter, preferably for fine dust, e.g. PM10 or PM2,5.

Preferably, said filter 521 is located upstream of said at least one fan 56 along the path of the air flow between said inlet 52 and said outlet 54.

More generally, in lamp 2 according to the present invention said inlet 52 is preferably positioned higher than said outlet 54, with reference to a vertical axis "Z". The present solution permits taking in air from a higher position, where dust concentration is lower. Such a solution results in a longer life of the filter 521, thus reducing the costs incurred for maintaining lamp 2.

More generally, in lamp 2 according to the present invention said ozonizer 6 is of the corona-effect type. The present solution ensures effective and inexpensive ozone production. Preferably, said ozonizer 6 can generate at least 20 g/h of ozone. Even more preferably, said ozonizer 6 can produce 20 g/h to 40 g/h of ozone.

Preferably, said ozonizer 6 can be programmed to produce different ozone quantities according to specific requirements, e.g. depending on the dimensions of the space "V" where said lamp 2 is to be installed.

More generally, said lamp 2 can, as a function of the volume of space "V", produce a certain quantity of ozone for as long as necessary to completely sanitize space "V", e.g. at least 20 minutes, by distributing the ozone quickly and evenly, so that said space "V" can be sanitized within 1h to 2h, e.g. 1,5h, considering also the time necessary for ozone decay to occur.

In a preferred, but merely illustrative and non-limiting, embodiment of said lamp 2 according to the present invention, said outlet 54 is positioned lower than said inlet 52, with reference to said vertical axis "Z". The present solution makes it possible to exploit the greater specific weight of ozone, compared with air, so that said ozone will be distributed starting from the bottom and then will rise until it saturates the volume of space "V". The present solution makes it possible to start the sanification process from the surfaces, in particular from those surfaces that require a more intense sanification, particularly the floor, the furniture, and all objects and/or accessories that are most likely to come in contact with users, thus being potentially and more easily contaminated.

Preferably, said outlet 54 is defined by a plurality of holes, through which air enriched with ozone produced by said ozonizer 6 can exit.

In a preferred, but merely illustrative and non-limiting, embodiment of said lamp 2 according to the present invention, said sanification device 5 comprises an ozone sensor 57 adapted to detect the presence of ozone.

With the present solution it is possible to check, during the production of ozone by sanification device 5, if the quantity of ozone in space "V" has reached the desired level. Moreover, said solution permits monitoring the reduction progressively occurring in the ozone level after said sanification device 5 has stopped producing ozone 5, e.g. in order to determine when space "V" can be accessed again.

Said ozone sensor 57 is electrically and electronically connected to said control system 58, thus allowing said control system 58 to receive the data generated by said ozone sensor 57.

Preferably, whenever a sanification cycle is started following the activation of sanification device 5, through said ozone sensor 57 it is possible to detect the set saturation level and/or the ozone saturation time in space "V", in particular by processing the data generated by said ozone sensor 57 by means of said control system 58. Preferably, said control system 58 comprises a timer in order to be able to determine how long a predetermined ozone level, e.g. saturation, has been maintained, so as to be able to determine the efficacy of the sanification of space "V", in particular of the surfaces in space "V". By way of example, depending on the elements that need to be abated, such as bacteria, viruses, insects, moulds, etc., predetermined ozone levels will have to be maintained for predetermined time intervals. In particular, in order to guarantee the sanification effect, in particular on the surfaces of space "V", ozone quantity and sanification time will vary according to the elements to be abated.

Preferably, when said sanification cycle is complete, said control system 58 uses the data generated by said ozone sensor 57 to determine the value of ozone decay in space "V", in particular the transformation from O₃ to O₂. In particular, upon reaching a predetermined threshold considered to be healthy for humans, e.g. approx. 150 ppb, control system 58 will provide feedback about the possibility of safely entering said space "V".

More generally, said control system 58 is adapted to control the activation of sanification device 5, in particular by appropriately activating said ozonizer 6 and said fan 56.

Said control system 58 comprises a computing unit, e.g. a microcontroller, capable of processing the data for the purpose of monitoring and controlling sanification device 5. For example, said control system 58 can receive commands for activating or deactivating sanification device 5, in addition to receiving the data concerning the ozone quantity detected by said ozone sensor 57. Also, said control system 58 is adapted to send commands for activating or deactivating said ozonizer 6 and/or said fan 56. By way of example, said control system 58 is adapted to suitably turn on said fan 56, even when said ozonizer 6 is off, in order to speed up ozone decay by moving the air in space "V". This solution ensures that space "V" will become accessible again sooner after a sanification cycle.

In a preferred embodiment of lamp 2 according to the present invention, said control system 58 comprises a safety system applied to the power supply circuit of said sanification device 5, and particularly to the power supply of said ozonizer 6. The present embodiment ensures a higher degree of safety in case of malfunctions or faults.

In one possible embodiment of said safety system, a first pole of the power supply circuit is managed, via electronic activation, by the microcontroller of control system 58; while a second pole of the power supply circuit is managed by a second controller, different from said microcontroller of control system 58, adapted to activate a relay with a normally open contact. This embodiment can prevent errors in activating ozonizer 6 or undesired accidental activations of the same, e.g. due to malfunctions or faults.

In one possible embodiment of lamp 2 according to the present invention, it comprises a dispenser device (not shown). Said dispenser device comprises, in turn, a container adapted to contain a substance, in particular in the liquid and/or gaseous state; and a dispenser element, adapted to take a known quantity of substance from said container and dispense said substance into space "V". Said dispenser element is adapted to spray, e.g. jet-spray, and/or nebulize said substance taken from said container.

Said dispenser device is electronically connected to said control system 58, the latter being adapted to control the activation of said dispenser device and possibly also to signal the presence, or the absence, of substance in said container. Preferably, said dispenser device is activated by control system 58, e.g. at the end of every sanification cycle. The activation of the dispenser device by means of said control system 58 may also occur independently of sanification device 5, e.g. at predefined time intervals, e.g. at regular intervals, or upon detection of the presence of a user in said space.

With the present solution, ozone decay can be made to occur more quickly in space "V".

In one possible embodiment of lamp 2 according to the present invention, said substance contained in the container of the dispenser device comprises at least one essence, e.g. a fragrance and/or a perfume, and/or a repellent, e.g. a mosquito repellent. The present solution ensures, in addition to the effect of perfuming the space or repelling insects such as mosquitoes, a considerable reduction in the time necessary for the ozone in space "V" to decay, thus shortening the time one needs to wait before one can safely enter space "V" and stay therein. In one possible embodiment, said container is a refillable container, or else it is a refill container to be replaced when empty. Said dispenser element may be embodied in several possible forms, e.g. depending on the type of substance to be dispensed and/or the type of container used in the dispenser device.

Describing now in more detail the construction of lamp 2 according to the present invention, in a preferred embodiment thereof said sanification device 5 is incorporated into said support structure 24. This solution allows said inlet 52 and said outlet 54 to be positioned furthest from each other, in particular along said vertical axis "Z".

In a first alternative embodiment of lamp 2 according to the present invention, said sanification device 5 is incorporated into said base 22. This solution allows said sanification device 5 to be enclosed into a structure that can be laid on a surface of space "V", while also ensuring stability of lamp 2. In fact, positioning the various components of sanification device 5 in said base 22 results in greater stability of said lamp 2, preventing it from falling.

In another alternative embodiment of lamp 2 according to the present invention, said sanification device 5 is incorporated into said lampshade 4, or at least into a portion thereof. This solution is advantageous in those embodiments wherein said lamp 2 is designed to be secured to vertical surfaces, e.g. walls, and/or to ceilings of space "V".

According to further alternative embodiments of lamp 2 according to the present invention, said sanification device is incorporated into two or more parts of lamp 2, including said base 22, said support structure 24 and said lampshade 4.

More generally, in lamp 2 according to the present invention said inlet 52 is preferably positioned higher than said outlet 54, with reference to said vertical axis "Z". This solution exploits the greater specific weight of ozone compared with that of air, so that said ozone will be emitted starting from a low height and will then rise until it saturates the volume of space "V", in addition to the fact that air is taken in from a region where dust concentration is lower.

In a preferred, but merely illustrative and non-limiting, embodiment of lamp 2 according to the present invention, said control system 58 comprises a wireless connection system 582. Said wireless connection system 582 is adapted to connect to a remote system, e.g. a centralized system 1, to allow said sanification device 5 to be activated and/or controlled remotely.

In one possible embodiment, said wireless connection system 582 comprises a transceiver system based on Bluetooth technology. The present solution turns out to be particularly useful for activating a lamp 2, in particular only one, from a remote position, e.g. by means of another device equipped with a suitably programmed transceiver system based on Bluetooth technology, e.g. a smartphone, which can be used outside the space "V" to turn on said sanification device 5. This solution also allows a user standing outside space "V" to check the operation of said sanification device 5, e.g. to check or monitor the sanification procedure, e.g. to determine if said ozonizer 6 is still in operation and/or if it is already safe to enter said space "V", e.g. by monitoring the data generated by said ozone generator 57.

In one possible embodiment, said wireless connection system 582 comprises a transceiver system based on Wi-Fi technology. This solution turns out to be particularly useful to create a network of lamps 2 positioned in different spaces "V" of the same building "E", so that through a centralized system it is possible to know the operating state of each lamp 2, and therefore to know the sanification state of each space "V" and whether or not each space "V" is accessible. This solution also makes it possible to activate each individual lamp 2, through said centralized system 1, for the purpose of sanitizing the corresponding space "V" in building "E", and to come to know of any malfunctions of one or more lamps 2 comprised in the network of lamps 2.

Said centralized system 1 is, for example, a control system adapted to control one or more parameters and/or variables associated with a plurality of spaces "V" of a building "E".

More generally, said building "E" may be a house comprising a plurality of rooms, each one defining a space "V", or a hospitality building, e.g. a hotel, comprising a plurality of rooms, each one comprising at least one space "V".

In a preferred embodiment of lamp 2 according to the present invention, said lamp 2 comprises a remote control 12. Said remote control 12 is adapted to control said sanification device 5, and particularly said control system 58, for activating and deactivating said ozonizer 6 and preferably also said fan 56. Preferably, said remote control 12 is of the optical type, to ensure the activation of a single sanification device 5 comprised in that specific lamp 2 which is comprised in space "V" where said remote control 12 is located.

More generally, in lamp 2 according to the present invention said power supply circuit 20 comprises, advantageously, a switch 21. Said switch 21 is adapted to interrupt the supply of power to said source 3, in particular to said source 3 only, while ensuring that power is still supplied to said sanification device 5. Said switch 21 does not affect the power supply to said sanification device 5. Therefore, even when source 3 is receiving no power because switch 21 is off, said sanification device 5 will still be operable.

Preferably, said lamp 2 is designed in such a way that when said lamp 2 is activated for illuminating space "V", i.e. when said source 3 is on, said sanification device 5 is off and cannot be turned on. It is assumed, in fact, that an observer or guest is in space "V" when source 3 is on, and it is therefore necessary to prevent the sanification cycle from starting when people are present in space "V". Moreover, in the embodiment wherein sanification device 5 can be activated by means of said remote control 12, the actual production of ozone by ozonizer 6 is delayed, e.g. for a period of time of 30 seconds to 3 minutes, in order to allow the person who has activated sanification device 5 to exit space "V".

More generally, depending on source 3 in use, said power supply circuit 20 is designed to allow source 3 to be correctly activated in order to emit light in the visible spectrum for illuminating space "V".

Said power supply circuit 20 is, for example, adapted to conduct 240V or 110V alternating current, e.g. coming from a source like a domestic and/or industrial power distribution network.

As a function of source 3 employed in lamp 2, said power supply circuit may comprise transformers, stabilizers and/or rectifiers to appropriately supply power to said source 3, e.g. when LED sources are used.

More generally, said switch 21 may be an on-off pushbutton, or it may operate as a dimmer to adjust the intensity of the light emitted by said source 3.

Said base 22 may have any shape and size, depending on the embodiment of said lamp 2, in particular to ensure the necessary stability of lamp 2, and also on the aesthetic design of said lamp 2, so as to perform also an ornamental and aesthetic function. Furthermore, said base 22 may be made of different materials, e.g. plastic, metal, wood, glass, etc., according to technical/structural and aesthetic requirements.

Said support structure 24 may have any shape and size, depending on the embodiment of said lamp 2, and particularly as a function of the distance to be obtained from said base 22, specifically the type of lamp 2 (table lamp, column lamp, wall light, ceiling light fixture, chandelier, etc.), and also on the aesthetic design of said lamp 2, so as to perform also an ornamental and aesthetic function. Furthermore, said support structure 24 may be made of different materials, e.g. plastic, metal, wood, glass, etc., according to technical/structural and aesthetic requirements.

Said lampshade 4 may have any shape and size, depending on the embodiment of said lamp 2, and particularly to avoid direct illumination towards an observer according to the type of lamp 2 (table lamp, column lamp, wall light, ceiling light fixture, chandelier, etc.), and also on the aesthetic design of said lamp 2, so as to perform also an ornamental and aesthetic function. Furthermore, said lampshade 4 may be made of different materials, e.g. fabric and/or plastic, metal, wood, glass, crystal, etc., according to technical/structural and aesthetic/ornamental requirements.

In one possible, but merely illustrative and non-limiting, embodiment, said lamp 2 has a height along said vertical axis "Z" of approximately 60cm, with base 22 and/or support structure 24 being approximately 15cm wide and 10cm deep.

Said source 3 is connected to a bulb socket 32, which is mechanically fixed to said support structure 24.

Source 3 may be an incandescent, halogen, fluorescent, fluorescent-tube, NEON, metal-iodide, magnetic-induction, LED, etc. source.

Said bulb socket 32 is designed in accordance with the type of source 3 in use, and in compliance with the standard on which said source 3 is based, e.g. the Edison standard or, more generally, any industrial standard of said sources 3 *per se* known to those skilled in the art.

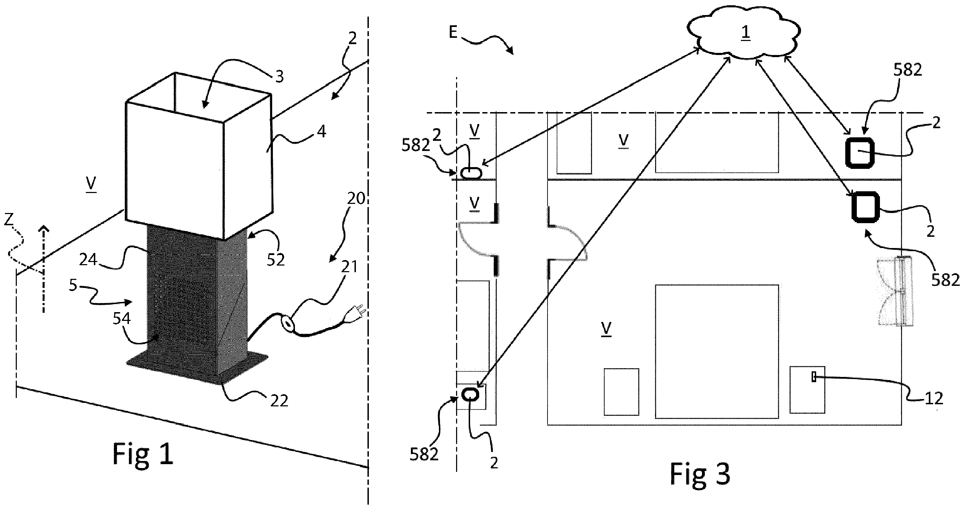

Figure 1 shows, by way of non-limiting example, one possible embodiment of lamp 2 according to the present invention, into which a sanification device 5 is integrated. Figure 1 shows a table lamp 2 for illuminating at least one space "V" of a building, e.g. a hotel room. In Figure 1 one can see that there is at least one source 3, adapted to emit light in the visible spectrum for illuminating said space "V", which source is hidden to the eye by a lampshade 4 adapted to divert the light emitted by source 3, e.g. in order to create a soft light and reduce the intensity of the light of source 3 perceivable by an observer.

The drawing also shows a power supply circuit 20, adapted to supply power to said source 3, which circuit comprises a switch 21.

Figure 1 shows also a base 22 adapted to be laid on a surface available in said space "V". Said surface is, for example, a table, a piece of furniture and/or a writing desk that is present in space "V".

A support structure 24 is structurally connected to, and extends from, base 22. Said support structure 24 is adapted to support said source 3. To said support structure 24 said lampshade 4 is also secured.

In the present embodiment of lamp 2 according to the present invention, sanification device 5 for sanitizing said space "V" is integrated into said support structure 24 of lamp 2. Though not shown in the drawing, it is clear that said sanification device 5 receives power from said power supply circuit 20.

Figure 1 also shows that said sanification device 5 comprises an inlet 52, adapted to allow air to enter, and an outlet 54, adapted to allow air to exit. Between said inlet 52 and said outlet 54 an ozonizer is positioned (not shown).

In the embodiment shown in Figure 1, said inlet 52 is positioned higher than said outlet 54, with reference to said vertical axis "Z".

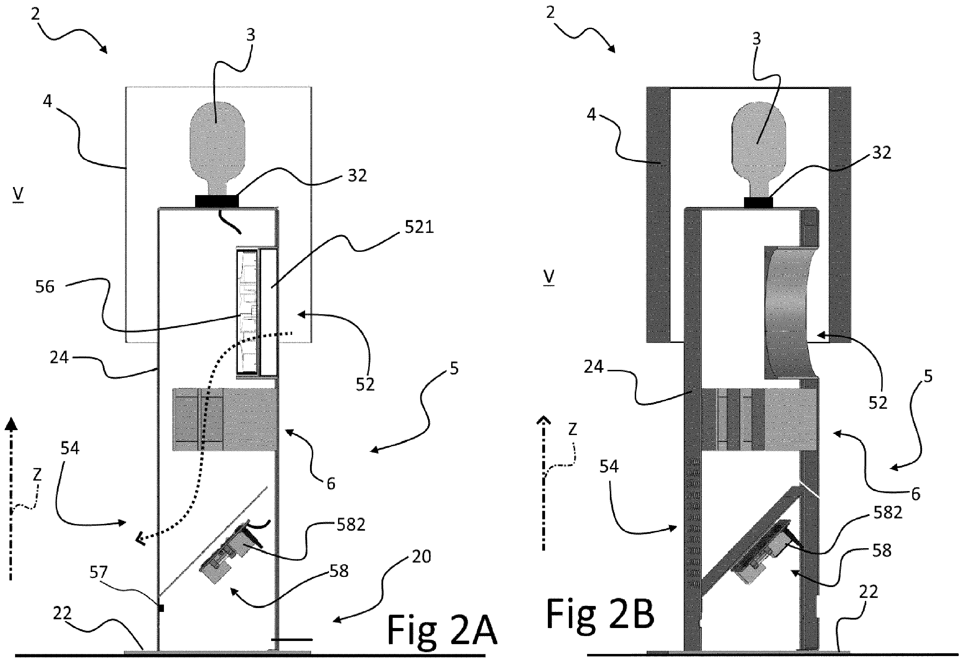

Figure 2A shows a sectional view of one possible embodiment of lamp 2, e.g. lamp 2 of Figure 1.

The figure shows that lampshade 4 covers source 3, provided in the form of a bulb, thus hiding it to the observer's eye. Said source 3 is fixed to a respective bulb socket 32, which may be constructed in compliance with the Edison standard.

Said source 3 is powered by said power supply circuit 20, which is only partially visible.

According to this embodiment, sanification device 5 is incorporated into support structure 24, which supports both source 3 and lampshade 4.

Down along said vertical axis "Z" inlet 52 is visible, which is formed in support structure 24. Said inlet 52 defines a housing for both a removable filter 521 and a fan 56, which sucks in air from space "V" and directs such air along the path indicated by the dotted line.

Underneath said inlet 52 there is a corona-effect ozonizer 6, suitably housed within a protection enclosure.

Underneath said ozonizer 6 there is said outlet 54, formed in support structure 24, through which the air saturated with ozone generated by ozonizer 6 flows out to saturate said space "V".

Sanification device 5 is also powered by power supply circuit 20, although the latter is only partially shown in the drawing.

Figure 2A also shows, arranged in proximity to base 22, control system 58, which comprises a wireless connection system 582, and an ozone sensor 57, adapted to detect the presence of ozone in space "V". Said base 22 is adapted to be laid on a surface available in the space "V", e.g. a table, a shelf, etc.

Figure 2B shows another possible embodiment of lamp 2 in a different sectional view, e.g. a rotated one. In this figure one can see that lampshade 4 covers source 3, provided in the form of a bulb, thus hiding it to the observer's eye. Said source 3 is fixed to a respective bulb socket 32, e.g. compliant with the Edison standard. Said source 3 is powered by a power supply circuit (not shown).

According to this embodiment, sanification device 5 is incorporated into support structure 24, which supports both source 3 and lampshade 4.

Down along said vertical axis "Z" inlet 52 is visible, which is formed in support structure 24. The figure shows that said inlet 52 defines at least one housing, e.g. adapted to receive a removable filter and/or a fan.

Underneath said inlet 52 there is a corona-effect ozonizer 6, suitably housed within a protection enclosure.

Underneath said ozonizer 6 there is said outlet 54, formed in support structure 24 as a plurality of holes, through which the air saturated with ozone generated by ozonizer 6 flows out to saturate said space "V".

Figure 2B also shows, arranged in proximity to base 22, control system 58, which comprises a wireless connection system 582. Although not shown, there are also an ozone sensor, adapted to detect the presence of ozone in space "V", and a power supply circuit, which is the same circuit that supplies power to said source 3. Said base 22 is adapted to be laid on a surface available in space "V", e.g. a table, a shelf, etc.

Lastly, Figure 3 shows a plan view of a portion of a building "E" in which there are a plurality of spaces "V", in each one of which there is at least one lamp 2 according to the present invention.

Figure 3 shows four spaces "V", suitably arranged and respectively accessible through an opening, in particular a door (shown for two spaces "V" only). Said spaces "V" are, for example, hotel rooms, each one comprising also a window.

The figure also shows four lamps 2, one for each space "V".

In this figure one can see that the lamps may have different dimensions and may be placed in different locations, e.g. on the floor (floor or column lamp) or on a piece of furniture (table lamp or bedside lamp), or fixed to a wall, e.g. a vertical wall or a ceiling (chandelier, ceiling light fixture or wall light.

Each lamp 2 according to the present invention shown in Figure 3 comprises a wireless connection system 582, e.g. based on Wi-Fi technology, which allows each lamp 2 to connect to a centralized system 1, the latter being capable of receiving data from each lamp 2, e.g. data about the operating state of the sanification device 5 and the ozone level in space "V", in addition to being able to send commands to each individual lamp 2, e.g. activation or deactivation commands.

In the embodiment illustrated in Figure 3, in a space "V" there is a lamp 2, which includes a sanification device that can be activated by means of a remote control 12.

More generally, lamp 2 according to the present invention can ensure effective sanification and disinfestation of all the surfaces that are present in a space "V" and also of the air that is present in said space "V".

The present invention does not require the execution of any works for positioning and installing the sanification device in a space of a building. In fact, the present invention only requires that the lamp be connected to a power supply circuit, such as an electric outlet of an electric circuit.

The present invention makes it possible to camouflage an effective sanification device into an object that is commonly present in a space of a building, such as a lamp.

The present invention results in a considerable cost reduction compared with the solutions currently known in the art, both from a construction viewpoint, since no works are necessary for positioning and installation, and from an amortization viewpoint, since it is incorporated into a furniture accessory that is commonly present in the spaces "V", so that, unlike the prior art, it does not require the purchase of two distinct and specific devices.

In addition to providing sanification of space "V", the present solution also permits reducing the dust that is present in space "V", because said filter 521 ensures dust filtration, thus making the air healthier.

Any alternative embodiments of lamp 2 which have not been described in detail herein, but which will be apparent to a person skilled in the art in light of the contents of the present patent application, shall be considered to fall within the protection scope of the present invention.

### REFERENCE NUMERALS

| | |
|---|---|
| Centralized system | 1 |
| Remote control | 12 |
| Lamp | 2 |
| Power supply circuit | 20 |
| Switch | 21 |
| Base | 22 |
| Support structure | 24 |
| Source | 3 |
| Bulb socket | 32 |
| Lampshade | 4 |
| Sanification device | 5 |
| Inlet | 52 |
| Filter | 521 |
| Outlet | 54 |
| Fan | 56 |
| Ozone sensor | 57 |
| Control system | 58 |
| Wireless connection system | 582 |
| Ozonizer | 6 |
| Building | "E" |
| Space | "V" |
| Vertical axis | "Z" |

## Claims

1. Lamp (2) for illuminating at least one space (V) of a building (E), comprising:
- at least one source (3) adapted to emit light in the visible spectrum for illuminating said space (V);
- at least one power supply circuit (20), adapted to supply power to said source (3);
- at least one base (22) adapted to be laid on a surface available in said space (V);
- at least one support structure (24), structurally connected to and extending from the base (22), and adapted to support said source (3);
- at least one lampshade (4) adapted to divert and/or create a soft light in order to reduce the intensity of the light of the source (3) perceivable by an observer;
- a sanification device (5) for sanitizing the surfaces and the air in said space (V);
said sanification device (5) being integrated into at least a part of said lamp (2) and being powered by said power supply circuit (20);
said sanification device (5) comprising:
- an ozonizer (6) adapted to generate ozone; and
- a control system (58) adapted to suitably activate said ozonizer device (6) independently of the activation of said source (3) of the lamp (2);
- at least one fan (56);
said control system (58) being adapted to activate said at least one fan (56) and said ozonizer (6) in a coordinated manner in order to maximize the distribution of ozone in said space (V);
said sanification device (5) comprises an ozone sensor (57) adapted to detect the presence of ozone; said ozone sensor (57) being electrically and electronically connected to said control system (58), so that said control system (58) can receive the data generated by said ozone sensor (57).

2. Lamp (2) according to claim 1, wherein said sanification device (5) comprises:
- at least one inlet (52) adapted to allow air to enter;
- at least one outlet (54) adapted to allow ozone-containing air to exit;
said ozonizer (6) being positioned between said inlet (52) and said outlet (54).

3. Lamp (2) according to claim 2, wherein said fan (56) can move 100 m³/h to 400 m³/h of air;
said fan (56) is located near the inlet (52), for the purpose of maximizing the air moving capacity.

4. Lamp (2) according to one of the preceding claims, wherein said ozonizer (6) is of the corona-effect type, adapted to generate at least 20 g/h of ozone.

5. Lamp (2) according to one of the preceding claims, wherein said sanification device (5) is enclosed in said support structure (24).

6. Lamp (2) according to claim 2, wherein said inlet (52) is positioned higher than said outlet (54), with reference to a vertical axis (Z).

7. Lamp (2) according to claim 1, wherein said control system (58) comprises a wireless connection system (582) adapted to connect to a centralized system (1) to allow said sanification device (5) to be activated and/or controlled remotely.

8. Lamp (2) according to claim 1 or 7, wherein said lamp (2) comprises a remote control (12) adapted to control said sanification device (5), and in particular said control system (58), for activating and deactivating said ozonizer (6) .

9. Lamp (2) according to claim 2, wherein said inlet (52) comprises at least one filter (521) of the removable type for fine-dust.

10. Lamp (2) according to claim 1, wherein said power supply circuit (20) comprises a switch (21) adapted to interrupt the supply of power to said source (3); said source (3) being connected to a bulb socket (32) mechanically fixed to said support structure (24).

11. Lamp (2) according to claim 7, wherein said wireless connection system (582) comprises a transceiver system based on Wi-Fi technology, thereby generating a network of lamps (2) located in different spaces (V) of a same building (E), said centralized system (1) being adapted to provide information about the operating status of each lamp (2), the sanification state of each space (V), and the accessibility of each individual space (V).

12. Lamp (2) according to claim 1, wherein said control system (58) comprises a timer for determining how long a predetermined ozone level has been maintained, for the purpose of determining the degree of sanification of the space (V).

13. Lamp (2) according to claim 1 or 12, wherein said control system (58) is adapted to determine the value of ozone decay in the space (V).

14. Lamp (2) according to one of the preceding claims, wherein said control system (58) comprises a safety system applied to the power supply circuit of said sanification device (5).

15. Lamp (2) according to one of the preceding claims, which comprises a dispenser device comprising:
- a container adapted to contain a substance; and
- a dispenser element, adapted to take a known quantity of substance from said container and dispense said substance in the space (V);
said dispenser element being adapted to spray and/or nebulize said substance taken from said container;
said dispenser device is electronically connected to said control system (58), the latter being adapted to control the activation of said dispenser device.
